(19) Europäisches Patentamt
European Patent Office
Office européen des brevets

(11) EP 3 895 693 B1

(12) EUROPEAN PATENT SPECIFICATION

(45) Date of publication and mention of the grant of the patent:
**09.10.2024 Bulletin 2024/41**

(21) Application number: **21167820.6**

(22) Date of filing: **12.04.2021**

(51) International Patent Classification (IPC):
***A61K 47/32*** *(2006.01)* ***A61K 47/34*** *(2017.01)*
***A61M 35/00*** *(2006.01)*

(52) Cooperative Patent Classification (CPC):
**A61K 9/0053; A61F 9/0017; A61K 47/32; A61K 47/34**

# (54) HYDROGEL COMPOSITION AND HYDROGEL LENS

HYDROGELZUSAMMENSETZUNG UND HYDROGELLINSE

COMPOSITION D'HYDROGEL ET LENTILLE D'HYDROGEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.04.2020 TW 109112349**
**25.11.2020 TW 109141266**

(43) Date of publication of application:
**20.10.2021 Bulletin 2021/42**

(73) Proprietor: **Pegavision Corporation**
**Taoyuan City 333 (TW)**

(72) Inventors:
- **WU, Hsin-Yi**
  **333 Taoyuan City (TW)**
- **LEE, Chong-Wei**
  **333 Taoyuan City (TW)**
- **CHEN, Chun-Han**
  **333 Taoyuan City (TW)**
- **CHANG, Han-Yi**
  **235 New Taipei City (TW)**

(74) Representative: **Viering, Jentschura & Partner mbB**
**Patent- und Rechtsanwälte**
**Am Brauhaus 8**
**01099 Dresden (DE)**

(56) References cited:
**EP-A1- 2 947 104** **CN-A- 101 397 347**

- **TANAKA Y ET AL: "Novel hydrogels with excellent mechanical performance", PROGRESS IN POLYMER SCIENCE, PERGAMON PRESS, OXFORD, GB, vol. 30, no. 1, 1 January 2005 (2005-01-01), pages 1 - 9, XP027691308, ISSN: 0079-6700, [retrieved on 20050101]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

This application claims the benefit of priority to Taiwan Patent Application Nos. 109112349, filed on April 13, 2020, and 109141266, filed on November 25, 2020.

## FIELD OF THE DISCLOSURE

The present disclosure relates to a hydrogel composition, and more particularly to a hydrogel composition and a hydrogel lens, which can load and slowly release active ingredients.

## BACKGROUND OF THE DISCLOSURE

EP 2947104 A1 discloses a composition for soft materials, which enables production of a soft material excellent in transparency, a stress relaxation property, and strength and having an elongation property that is not so much lowered even at high temperatures. This patent also aims to provide a soft material produced using the composition for soft materials of this patent. This patent relates to a composition for soft materials including polyrotaxane and a radical polymerizable monomer, the polyrotaxane including a cyclic molecule, a linear molecule threading through a cavity of the cyclic molecule in a skewered manner, and capping groups that cap both ends of the linear molecule, the polyrotaxane having at least one cyclic molecule with a radical polymerizable group, the polyrotaxane having at least two radical polymerizable groups.

Tanaka Y et al. (academic literature "Novel Hydrogels with Excellent Mechanical Performance", Progress In Polymer Science, Pergamon Press, Oxford, GB, vol. 30, no. 1, January 1 2005, pages 1-9, XP027691308, ISSN: 0079-6700) discloses three kinds of novel hydrogels with excellent mechanical performance that have been developed, based on different concepts. Two of them exhibit high resistance to extension, up to 10-20 times the original length, by introducing special cross-linking structures. The third has a high modulus (sub-megapascal), with a failure compressive stress as high as 20 MPa, through a double network structure. In this article, the structural and mechanical features of these gels and the status of current studies have been described.

CN 101397347 B discloses an aquagel with high intensity, a preparation method and the function thereof. The method comprises the following steps of: 1) preparing an aqueous surfactant solution with certain concentration and conducting irradiation and overoxidation under the condition of oxygen entrance; 2) mixing the aqueous surfactant solution obtained from step 1) with monomers and distilled water according to a certain volume ratio, inletting nitrogen to deoxidize, and sealing the container to form a reaction system; and 3) putting the sealed reaction system under 30-100 DEG C to react for 0.5-48 hours to obtain the aquagel with high mechanical intensity. The aquagel is optically transparent and has high mechanical intensity, and the softness and hardness of which can be conveniently adjusted by controlling the preparation process, so as to meet the needs of different application occasions. The aquagel can be applied to biological, medical, optical devices and the like fields. The method of this patent is realized by low-temperature and normal-pressure operation, low dosage, low energy consumption, high production efficiency, simple process and low cost.

In the past decades, delivery of ophthalmic drugs (or active ingredients) has been an important challenge for ophthalmologists, and topical eye drops were commonly used to treat human eyes. However, in this way of drug delivery, the drug is instantly diluted instantly after being dropped into an eye of a human body, and can easily be discharged from a tear orifice of the eye. A retention time of the drug in the eye of the human body is short, which results in a poor therapeutic effect. Therefore, ophthalmologists may need to consider increasing a usage frequency or a dosage of the drug, but such measures may also increase risks of side effects from the drug. Therefore, the above-mentioned drug delivery method still has room for improvement.

## SUMMARY OF THE DISCLOSURE

In response to the above-referenced technical inadequacies, the present disclosure provides a hydrogel composition and a hydrogel lens according to independent claims 1 and 36. The dependent claims show further embodiment of claims 1 and 36.

In one aspect, the present disclosure provides a hydrogel composition which includes a hydrophilic monomer, a cross-linker, an initiator and a rotaxane compound. Based on a total weight of the hydrogel composition, a content range of the hydrophilic monomer is between 60 wt% and 99.85 wt%, a content range of the cross-linker is between 0.01 wt% and 1 wt%, a content range of the initiator is between 0.01 wt% and 2 wt%, and a content range of the rotaxane compound is between 0.1 wt% and 15 wt%. The rotaxane compound includes at least one cyclic molecule and at least one linear molecule passing through the at least one cyclic molecule in a string manner, and a weight ratio of the rotaxane compound relative to the hydrophilic monomer is between 1:6 and 1:99. In the rotaxane compound, a number average molecular weight of the linear molecule is between 2,000 and 20,000, and the rotaxane compound does not include any

slopper or capping used for preventing the cyclic molecule from detaching from two ends of the linear molecule.

In another aspect, the present disclosure provides a hydrogel lens which includes a lens body that is formed by the above-mentioned hydrogel composition.

Therefore, by virtue of "introduction of a rotaxane compound into a hydrogel composition", the hydrogel composition and the hydrogel lens of the present disclosure can have effects of loading and slowly releasing active ingredients.

It is worth mentioning that, although the rotaxane compound is introduced into the hydrogel lens, the hydrogel lens of the present disclosure can not only have the effects of loading and slowly releasing the active ingredients, but also maintain required characteristics of the hydrogel lens, such as a lens base curve (BC), a lens center thickness (CT), a lens diameter (DIA), a refractive index, a visible light transmittance, and a dynamic contact angle.

## BRIEF DESCRIPTION OF THE DRAWINGS

The described embodiments may be better understood by reference to the following description and the accompanying drawings, in which:

FIG. 1 is a schematic view showing a rotaxane compound dispersed in a hydrogel composition according to an embodiment of the present disclosure; and

FIG. 2 is a schematic view showing cyclic molecules stacked on top of each other according to the embodiment of the present disclosure.

## DETAILED DESCRIPTION OF THE EXEMPLARY EMBODIMENTS

The present disclosure is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a", "an", and "the" includes plural reference, and the meaning of "in" includes "in" and "on". Titles or subtitles can be used herein for the convenience of a reader, which shall have no influence on the scope of the present disclosure.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present disclosure or of any exemplified term. Likewise, the present disclosure is not limited to various embodiments given herein. Numbering terms such as "first", "second" or "third" can be used to describe various components, signals or the like, which are for distinguishing one component/signal from another one only, and are not intended to, nor should be construed to impose any substantive limitations on the components, signals or the like.

According to the technical inadequacies mentioned in the background of the present disclosure, in the past decades, delivery of ophthalmic drugs (or active ingredients) has been an important challenge for ophthalmologists, and topical eye drops were commonly used to treat human eyes. However, in this way of drug delivery, the drug is instantly diluted instantly after being dropped into an eye of a human body, and can easily be discharged from a tear orifice of the eye. A retention time of the drug in the eye of the human body is short, which results in a poor therapeutic effect. Therefore, ophthalmologists may need to consider increasing a usage frequency or a dosage of the drug, but such measures may also increase risks of side effects from the drug. Therefore, the above-mentioned drug delivery method still has room for improvement.

To improve the technical inadequacies mentioned above, an object of the present disclosure is to provide a new type of hydrogel lens (i.e., hydrogel contact lens), which is a strategy for transportation of active ingredients. That is, the present disclosure provides a new delivery mode of the active ingredients by using the new type of hydrogel lens, which can load and slowly release the active ingredients. The technology of the present disclosure can maintain the active ingredients on the hydrogel contact lens to prolong a retention time of the active ingredients on the human eyes, and improve a utilization efficiency of the active ingredients, thereby improving wearing comfort for a user, and even achieving a long-term maintenance effect.

[Hydrogel Composition]

To achieve the above object, an embodiment of the present disclosure provides a hydrogel composition for preparing a hydrogel lens. The hydrogel composition includes a hydrophilic monomer, a cross-linker, an initiator, and a

rotaxane compound.

Based on a total weight of the hydrogel composition being 100 wt%, a content range of the hydrophilic monomer in the hydrogel composition is between 60 wt% and 99.85 wt%, and preferably between 68.88 wt% and 99.85 wt%. A content range of the cross-linker in the hydrogel composition is between 0.01 wt% and 1 wt%, and preferably between 0.04 wt% and 0.57 wt%. A content range of the initiator in the hydrogel composition is between 0.01 wt% and 2 wt%, and preferably between 0.05 wt% and 0.72 wt%. In addition, a content range of the rotaxane compound in the hydrogel composition is between 0.1 wt% and 15 wt%, preferably between 0.1 wt% and 12 wt%, and more preferably between 0.5 wt% and 8 wt%.

To enable the rotaxane compound to be uniformly dispersed in the hydrogel composition and to exert an expected effect, a weight ratio of the rotaxane compound relative to the hydrophilic monomer is between 1:6 and 1:99, preferably between 1:11 and 1:99, and more preferably between 1:14 and 1:74.

If the weight ratio of the rotaxane compound relative to the hydrophilic monomer is less than a lower limit of the above-mentioned ratio range (i.e., 1:1), the rotaxane compound may not be uniformly dispersed in the hydrogel composition due to a high addition amount of the rotaxane compound, so that a finally formed hydrogel contact lens may have poor quality, such as poor refractive index or poor light transmittance.

On the contrary, if the weight ratio of the rotaxane compound relative to the hydrophilic monomer is greater than an upper limit of the above-mentioned ratio range (i.e., 1:200), the rotaxane compound may not be able to exert the expected effect in the finally formed hydrogel contact lens (i.e., effectively loading and slowly releasing the active ingredients) due to a low addition amount of the rotaxane compound.

Material types of each component in the hydrogel composition according to the embodiment of the present disclosure will be described in the following paragraphs.

The hydrophilic monomer preferably has a vinyl group, an acetyl group, a propenyl group, or an acryl group in its molecular structure.

In various embodiments of the present disclosure, the hydrophilic monomer is at least one material selected from a group consisting of N-vinyl pyrrolidone (NVP), 2-hydroxyethyl methacrylate (HEMA), methacrylic acid (MAA), methyl methacrylate (MMA), acrylic acid (AAc), N,N-dimethyl acrylamide (DMA), 2,3-dihydroxypropyl methacrylate (GMMA), N,N-dimethyl methacrylamide, and N-vinyl-N-methyl acetamide.

In various embodiments of the present disclosure, the hydrogel composition further includes a silicone compound, and the silicone compound is at least one material selected from a group consisting of silicon monomer, silicon-based polymer (i.e., siloxane), and silicon pre-polymer. Accordingly, the finally formed hydrogel contact lens can be a silicone hydrogel contact lens, but the present disclosure is not limited thereto.

In various embodiments of the present disclosure, the cross-linker is at least one material selected from a group consisting of ethylene glycol dimethacrylate, diethylene glycol dimethacrylate, triethylene glycol dimethacrylate, tetraethylene glycol dimethacrylate, allyl methacrylate, triethylene glycol dially ether, tetraethylene glycol dially ether, and 1,1,1-trimethylolpropane trimethacrylate.

In various embodiments of the present disclosure, the initiator is a photo-initiator, and a content range of the photo-initiator in the hydrogel composition is between 0.05 wt% and 0.72 wt%.

The photo-initiator is at least one material selected from a group consisting of bis(2,6-difluoro-3-(1-hydropyrro-1-yl)- phenyl)titanocene, phenyl-bis-(2,4,6-trimethylbenzoyl)-phosphine oxide, and 2-hydroxy-2-methyl-1-phenyl-1-porpanone.

Furthermore, the rotaxane compound includes at least one cyclic molecule and at least one linear molecule, and the at least one linear molecule passes through the at least one cyclic molecule in a string manner.

To enable the rotaxane compound to effectively load and slowly release active ingredients, a weight ratio of the cyclic molecule relative to the linear molecule has a specific range. For example, the weight ratio of the at least one cyclic molecule relative to the at least one linear molecule is preferably between 1:1 and 50:1, and more preferably between 5:1 and 35:1.

That is, in the rotaxane compound, a quantity of the at least one cyclic molecule is generally plural, a quantity of the linear molecule is generally one, and the at least one linear molecule passes through a plurality of cyclic molecules.

Accordingly, a sufficient space can be formed between the plurality of cyclic molecules and the linear molecule to load an effective amount of active ingredients. Furthermore, a dynamic structure can be formed between the plurality of cyclic molecules and the linear molecule, so that the rotaxane compound can achieve an effect of slowly releasing the active ingredient.

If the weight ratio of the cyclic molecule relative to the linear molecule exceeds the above ratio range, the effect of the rotaxane compound on loading and slowly releasing the active ingredients may become unsatisfactory.

In terms of material types, the cyclic molecule may be, for example, cyclodextrin or a derivative thereof, but the present disclosure is not limited thereto.

In various embodiments of the present disclosure, the cyclodextrin is at least one material selected from a group consisting of α-cyclodextrin, β-cyclodextrin, γ-cyclodextrin, hydroxypropyl-β-cyclodextrin, (2-hydroxypropyl)-γ-cyclodextrin, sulfobutylether-β-cyclodextrin, and methyl-β-cyclodextrin.

In addition, the cyclic molecule may also be, for example, crown ether or a derivative thereof.

In various embodiments of the present disclosure, the crown ether is at least one material selected from a group consisting of 12-crown-4, 15-crown-5, 18-crown-6, benzo-18-crown-6, benzo-15-crown-5, dicyclohexyl-18-crown-6, 2-(hydroxymethyl)-12-crown-4-ether, 2-(hydroxymethyl)-15-crown-5-ether, and 2-(hydroxymethyl)-18-crown-6-ether.

Furthermore, the linear molecule is at least one material selected from a group consisting of polyethylene glycol (PEG), polyvinyl alcohol (PVA), and polypropylene glycol (PPG).

The polyethylene glycol has a chemical structure of formula (1):

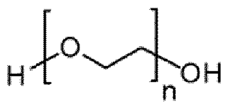

(1).

The polyvinyl alcohol has a chemical structure of formula (2):

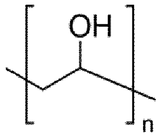

(2).

The polypropylene glycol has a chemical structure of formula (3):

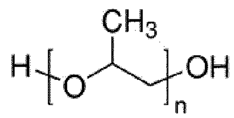

(3).

In addition, "n" is preferably a positive integer greater than 4, and more preferably a positive integer between 100 and 6,000. Furthermore, a number average molecular weight of the linear molecule is preferably between 200 and 20,000, but the present disclosure is not limited thereto.

It is worth mentioning that an object of the present disclosure is to introduce the "rotaxane compound" into the hydrogel composition that is used to prepare the hydrogel lens, so that the hydrogel lens can be cooperated with an inclusion technology and have the effect of loading and slowly releasing the active ingredient by the rotaxane compound.

The above-mentioned "inclusion technology" is to embed the active ingredient (also called target substance) into the cyclic molecule, so that the active ingredient can be in a stable state. Furthermore, the cyclic molecule can be combined with the linear molecule to form the rotaxane compound, thereby achieving the purpose of slowly releasing the active ingredient.

On the other hand, the "inclusion technology" refers to a technology in which one molecule is embedded into a cavity structure of another molecule to form an inclusion compound. Among them, the inclusion compound is composed of a host molecule and a guest molecule. In addition, the host molecule has a relatively large cavity structure, which is sufficient to contain the guest molecule to form a molecular capsule.

In a specific embodiment of the present disclosure, the cyclic molecule of the rotaxane compound is cyclodextrin, and the linear molecule of the rotaxane compound is polyethylene glycol (PEG), but the present disclosure is not limited thereto.

Further, a molecular structure of the cyclodextrin has a tire-like shape. A secondary hydroxyl group on C-2 and C-3 atoms of each glucose residue is located at one end of the cyclodextrin and has a slightly larger diameter. A primary hydroxyl group on C-6 atom of each glucose residue is located at another end of the cyclodextrin and has a slightly smaller diameter. Therefore, an inside of the cyclodextrin is a hydrophobic cavity structure, which can provide a fat-soluble active ingredient, such as menthol or vitamin E acetate, to enter and to be loaded therein.

The following chemical structures are chemical structures of three main types of cyclodextrins.

| cyclodextrin types | chemical structure |
|---|---|
| α-cyclodextrin | 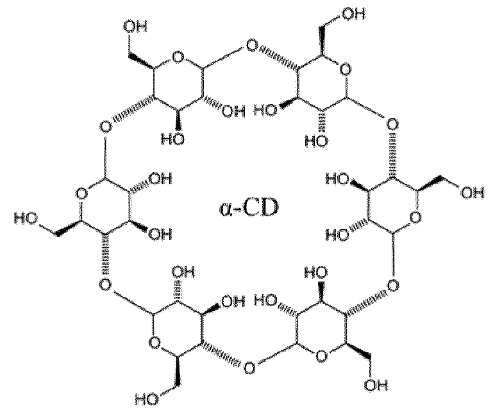 |
| β-cyclodextrin | 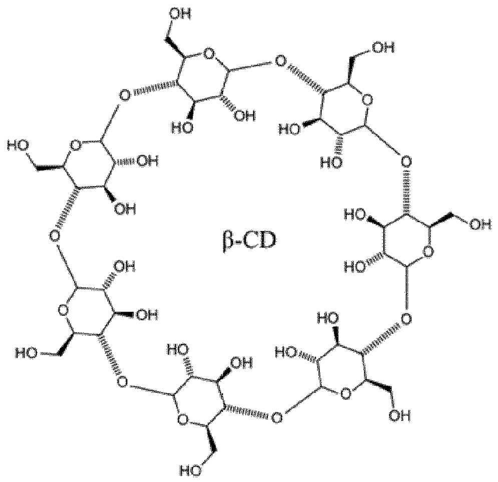 |
| γ-cyclodextrin | 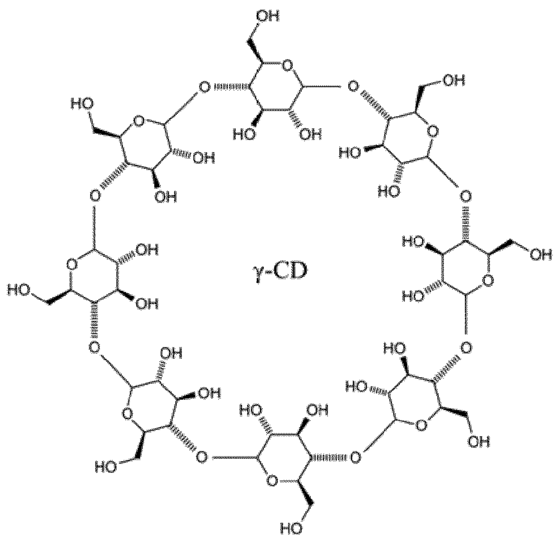 |

When a cyclodextrin and a fat-soluble active ingredient are dissolved in a solvent, the fat-soluble active ingredient usually enters a hydrophobic cavity structure of the cyclodextrin, a peripheral hydroxyl group (-OH group) of the cyclodextrin will interact with an aqueous solution or a highly-polar solution (i.e., hydrophilic monomer or contact lens care solution) to increase a solubility of the fat-soluble active ingredient.

In addition to forming a complex of the cyclodextrin and the fat-soluble active ingredient to increase the solubility of the fat-soluble active ingredient, the complex can be combined with a linear molecule (i.e., PEG) to form a rotaxane compound, thereby facilitating stable release of the loaded active ingredient.

More specifically, the above-mentioned rotaxane compound is formed by a supramolecular force between the cyclic molecule and the linear molecule. The rotaxane compound includes one or several cyclic molecules (wheel structures) and one linear molecule (shaft structure). The linear molecule and the cyclic molecules are connected by a weak interaction, such as mechanical bonds, instead of strong covalent bonds or coordination bonds. Therefore, this supramolecular polymer has less stability and a dynamic structure, which is suitable for loading an active ingredient, such as a small molecule drug, a protein drug, or a gene.

It is worth mentioning that for the present application, extensive research has been conducted on academic literature regarding the ability of the rotaxane compound to load active ingredients.

For example, the literature of Pol. J. Chem. Tech. 2016 18 (3) 110-116 pointed out that cyclodextrin can effectively load menthol; the literature of J fluoresc. 2007 17 265-270 pointed out that hydroxypropyl-β-cyclodextrin (HP-β-CD) can effectively load vitamin B12. In addition, the literature of J Agric Food Chem. 2017 65(26) 5404-5412 also pointed out that hydroxypropyl-β-cyclodextrin (HP-β-CD) can effectively load vitamin E, which can further increase the solubility of the vitamin E in water.

It is also worth mentioning that, as mentioned above, the weight ratio of the rotaxane compound relative to the hydrophilic monomer is usually between 1:6 and 1:99. In some specific embodiments of the present disclosure, a molecular structure of the hydrophilic monomer preferably has an acryl group, and in these specific embodiments, the weight ratio of the rotaxane compound relative to the hydrophilic monomer is preferably between 1:6 and 1:94.

The functional group having the acryl group may be, for example, at least one of the following four chemical structures.

| | |
|---|---|
| 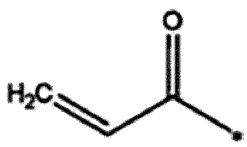 | 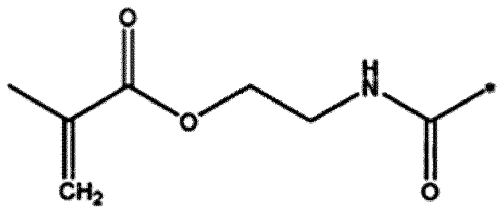 |
| 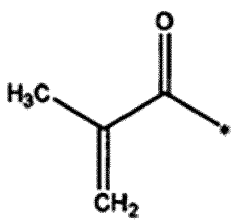 | 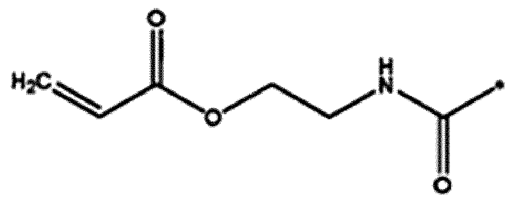 |

Furthermore, it has been found that a preferred range of the weight ratio of the rotaxane compound relative to the hydrophilic monomer is different under different material selection of cyclodextrin.

For example, when the cyclic molecule in the rotaxane compound is α-cyclodextrin or β-cyclodextrin, the weight ratio of the rotaxane compound relative to the hydrophilic monomer is preferably between 1:11 and 1:54. When the cyclic molecule in the rotaxane compound is hydroxypropyl-β-cyclodextrin, the weight ratio of the rotaxane compound relative to the hydrophilic monomer is preferably between 1:17 and 1:86. In addition, when the cyclic molecule in the rotaxane compound is (2-hydroxypropyl)-γ-cyclodextrin, the weight ratio of the rotaxane compound relative to the hydrophilic monomer is preferably between 1: 19 and 1:94.

To increase a ultraviolet light blocking ability of the hydrogel lens, in various embodiments of the present disclosure, the hydrogel composition further includes an ultraviolet light blocking monomer, and a content range of the ultraviolet light blocking monomer in the hydrogel composition is between 0.34 wt% and 1.68 wt%.

The ultraviolet light blocking monomer is at least one material selected from a group consisting of benzophenone

and benzotriazole.

To increase a solubility of the hydrogel composition, in various embodiments of the present disclosure, the hydrogel composition further includes a co-solvent, and a content range of the co-solvent in the hydrogel composition is between 4.33 wt% and 12.67 wt%.

The co-solvent is at least one material selected from a group consisting of glycerol, isopropyl alcohol, n-butanol, t-butanol, t-amyl alcohol, and n-hexanol.

To enable the hydrogel lens to have a specific color, in various embodiments of the present disclosure, the hydrogel composition further includes a dye, and a content range of the dye in the hydrogel composition is between 0.002 wt% and 0.039 wt%.

The dye is at least one material selected from a group consisting of reactive blue 19 (disodium 1-amino-9,10-dioxo-4-[3-(2-sulfonato oxyethyl sulfonyl) anilino] anthracene-2-sulfonate), Sudan III (1-[4-(phenylazo) phenylazo]-2-naphthol, Indigo (2,2'-bis(2,3-dihydro- 3-oxoindolyl idene)), and Quinoline Yellow (disodium 2-(1,3-dioxo- 2,3-dihydro-1H-inden-2-yl) quinolone-6,8-disulfonate).

Furthermore, the linear molecule (i.e., PEG, PVA, PPG) in the rotaxane compound has a relatively high molecular weight, and thus has a relatively long polymer chain. More specifically, the linear molecule in the rotaxane compound has a number average molecular weight between 2,000 and 20,000, preferably between 4,000 and 15,000, and more preferably between 4,000 and 8,000. According to the above configuration, since the linear molecule in the rotaxane compound has a sufficiently long polymer chain, the cyclic molecule has enough space to slide on the linear molecule, so that the cyclic molecule is not easily detached from the linear molecule. Accordingly, the rotaxane compound does not need to include any slopper or capping that is used to prevent the cyclic molecule from detaching from two ends of the linear molecule. In other words, in the rotaxane compound, the two ends of the linear molecule may not need to be formed with the slopper or capping, which can still stably maintain the shape of the rotaxane compound.

Furthermore, in the rotaxane compound, an outer periphery of the cyclic molecule (i.e., cyclodextrin) has a hydrophilic functional group (i.e., hydroxyl group, -OH group), so that the outer periphery of the cyclic molecule has a higher hydrophilicity. Furthermore, an inner side of the cyclic molecule has a hydrophobic cavity structure, so that the inner side of the cyclic molecule has a higher hydrophobicity, and the hydrophobic cavity structure can be used to provide fat-soluble active ingredients to enter therein, thereby enhancing a loading effect of the active ingredients. Since the outer periphery of the cyclic molecule has the hydrophilic functional group, the rotaxane compound can interact with the hydrophilic monomer in the hydrogel composition (i.e., NVP, HEMA) through the hydrophilic functional group on the outer periphery of the cyclic molecule. Accordingly, the rotaxane compound can be uniformly dispersed in the hydrogel composition based on the interaction between polar molecules (cyclic molecule and hydrophilic monomer).

More specifically, as shown in FIG. 1, in the hydrogel composition 100, the linear molecule 11 of the rotaxane compound 1 is dispersed in the hydrophilic monomer 2 in a tortuous form. In addition, the rotaxane compound 1 further includes a plurality of cyclic molecules 12, and the plurality of cyclic molecules 12 are threaded onto the linear molecule 11 in a series connection.

It is worth mentioning that, in the hydrogel composition 100, the plurality of series-connected cyclic molecules 12 and the adjacent plurality of series-connected cyclic molecules 12 are capable of being attracted to each other by a hydrogen bonding force and arranged in a stacking manner (as shown in FIG. 2).

In addition, referring to FIG. 1 again, in the hydrogel composition 100, parts of the cyclic molecules 12 are not threaded onto the linear molecule 11, but scattered on an outside of the linear molecule 11, which exist as independent molecules, but the present disclosure is not limited thereto.

Further, as shown in FIG. 2, based on the hydrogen bonding force, the plurality of series-connected cyclic molecules 12 on the linear molecule 11 are arranged in a manner of heads to heads and tails to tails.

In addition, it is worth mentioning that, the hydrogel composition 100 can have a relatively excellent tensile property through the addition of the rotaxane compound 1. Specifically, the hydrogel lens made from the hydrogel composition 100 has an elongation between 250% and 380%, and more preferably between 320% and 380%.

[Method for Preparing Hydrogel Composition]

The method for preparing the rotaxane compound may include, for example, adding the cyclic molecule and the linear molecule into water with the weight ratio described in the above-mentioned embodiment; mixing and stirring for several hours until the reaction is completed to obtain a reaction solution; and then drying the reaction solution to obtain a rotaxane compound powder.

The method for preparing the hydrogel composition may include, for example, slowly adding the rotaxane compound powder, a cross-linker, and an initiator to a hydrophilic monomer, and mixing and stirring for several hours until the reaction is completed to obtain the hydrogel composition. That is, in this embodiment, the cyclic molecule and the linear molecule are formed into the rotaxane compound before preparing the hydrogel composition.

In addition, the method for preparing the hydrogel composition can also include, for example, slowly adding

the cyclic molecule, the linear molecule, the cross-linker, the initiator and other materials to the hydrophilic monomer, and mixing and stirring for several hours until the reaction is completed to obtain the hydrogel composition. In other words, in this embodiment, the cyclic molecule and the linear molecule are formed into the rotaxane compound while preparing the hydrogel composition.

[Method for Preparing Hydrogel Lens]

The method for preparing the hydrogel lens may include, for example, injecting the above hydrogel composition into a mold for preparing the hydrogel lens; and performing a curing molding process on the hydrogel composition to form a semi-finished dry film product of the hydrogel lens.

The method further includes immersing the semi-finished dry film product of the hydrogel lens in a buffer solution until the hydrogel lens swells (i.e., hydration process); filling the buffer solution in a packaging container; immersing the hydrogel lens in the buffer solution; and then sealing and sterilizing the hydrogel lens, thus completing the production of the hydrogel contact lens product.

The above-mentioned active ingredient (i.e., the small molecule drug, the protein drug or the gene) can be added or mixed into the material used to prepare the contact lens; or, for example, the active ingredient can be loaded onto the contact lens during demolding and swelling; or, for example, the active ingredient can be dissolved in the buffer solution for soaking the contact lens and then loaded on the contact lens. Accordingly, when a user attaches the contact lens to his eye, the active ingredient can be slowly released from the contact lens over time and be absorbed by his eye.

In other words, the method for adding or loading the active ingredient (i.e., the small molecule drug, the protein drug or the gene) into the rotaxane compound may include: adding the active ingredient during the preparation of the lens material, adding the active ingredient during the hydration of the lens, or adding the active ingredient in the buffer solution.

Under this slow-release system, the active ingredient that can be loaded onto the contact lens may be, for example, an ingredient with cooling or anti-itching effects, such as menthol, l-menthol, camphor, d-camphor, dl-camphor, borneol, d-borneol, or potassium chloride.

The active ingredient may also be, for example, an ingredient with an antiinflammatory effect, such as: lysozyme hydrochloride, dipotassium glycyrrhizinate, 6-aminocaproic acid, ε-aminocaproic acid, 6-aminohexanoic acid acid, zinc sulfate, vitamin B2, sodium azulene sulfonic acid, sulfamethoxazole, sulfamethoxazole sodium, or prano-profen.

The active ingredient may also be, for example, an ingredient with an effect of relieving symptoms of conjunctival hyperemia, such as naphazoline, naphazoline hydrochloride, tetrahydrozoline, tetryzoline, tetrahydrozoline hydrochloride, chlorobutanol, chlorobutanol hemihydrate, phenylephrine hydrochloride, or phenylephrine.

The active ingredient may also be, for example, an ingredient with antioxidation or eye fatigue effects, such as vitamin E, α-tocopheryl acetate, L-potassium aspartate, magnesium L-aspartate, chondroitin sulfate, chondroitin sulfate sodium salt, sodium chondroitin chloride, neostigmine methyl-sulfate, arginine, vitamin B12, cyanocobalamin, methylcobalamin, adeno-sylcobalamin, hydroxo-cobalamin, vitamin B5, pantothenic acid, vitamin B6, pyridoxine hydrochloride, vitamin A, vitamin A palmitate, or taurine.

The active ingredient may also be, for example, an ingredient with anti-allergic or anti-itch effects, such as diphenhydramine, diphenhydramine hydrochloride, chlorpheniramine, chlorpheniramine maleate, or tranilast

In addition, the active ingredient may also be, for example, an ingredient with moisturizing or soothing effects, such as boric acid, glycerine, hyaluronic acid, polyol, or allantoin.

[Hydrogel Lens]

The embodiment of the present disclosure also provides a hydrogel lens (hydrogel contact lens) including a lens body that is formed by the above hydrogel composition.

The rotaxane compound is distributed on at least one surface of a concave arc surface or a convex arc surface of the lens body to form a rotaxane layer, and the rotaxane compound is configured to carry an active ingredient. The active ingredient is at least one of the small molecule drug, the protein drug, and the gene.

Accordingly, when a user attaches the contact lens (hydrogel lens) to his eye, the active ingredient can be slowly released from the contact lens over time and be absorbed by his eye.

In addition, a refractive index of the lens body is preferably between 1.315 and 1.598, and more preferably between 1.398 and 1.485.

A visible light transmittance of the lens body is preferably not less than 90%, and more preferably not less than 95%.

A dynamic contact angle (DCA) of the lens body is preferably less than 38, and more preferably less than 35, thereby having good wettability.

In various embodiments of the present disclosure, the lens body of the contact lens may also be, for example,

any lens body known in the technical field. For example, the lens body may be, for example, a rigid gas permeable (RGP) contact lens, a hydrogel soft contact lens (SCL), or a silicone hydrogel soft contact lens.

[Experimental Data and Test Results]

Hereinafter, the content of the present disclosure will be described in detail with reference to Exemplary Examples 1 to 4 in Table 1. However, the following examples are only used to help understand the present disclosure, and the scope of the present disclosure is not limited to these examples.

The hydrogel lenses of the Exemplary Examples shown in Table 1 are obtained by the above preparation method of the hydrogel composition and the preparation method of the hydrogel lens.

After the preparations of the exemplary lenses in Table 1 are completed, physical and chemical properties are tested, such as: a base curve (BC), a center thickness (CT), a diameter (DIA), a refractive index, a light transmittance, a water content, an atmospheric dehydration rate, a lubricity, a dynamic contact angle (DCA), an elongation, a water extraction test, and a hexane extraction test.

The dynamic contact angle (DCA) is measured by using a dynamic contact angle measuring instrument, and a captive bubble method is used as the method to measure the dynamic contact angle. The captive bubble method is a measurement method often used to measure the wettability or hydrophilicity of contact lenses. Generally, the dynamic contact angle of a contact lens can be less than 50 degrees. According to the following examples 1 to 4, the dynamic contact angles of the hydrogel contact lenses are not more than 80 degrees, specifically not more than 38 degrees, and more specifically not more than 35 degrees. That is, the hydrogel contact lenses in the examples 1 to 4 all have good wettability.

The atmospheric dehydration rate is one of the important indicators that can be used to evaluate a water retention level of a lens material. The measurement method of the atmospheric dehydration rate is to: place a hydrated and swelled hydrogel lens on a damp cloth and gently wipe the hydrogel lens to remove excess water; put the hydrogel lens on a sample pan of a scale; weigh and record a weight of the hydrogel lens; and record the weight of the hydrogel lens at different time points, such as the $10^{th}$ minute of atmospheric dehydration, the $30^{th}$ minute of atmospheric dehydration, the $60^{th}$ minute of atmospheric dehydration. Table 1 shows the measurement results of the atmospheric dehydration rates of the lenses in different exemplary examples.

According to market research, rapid loss of moisture in contact lens is one of the main reasons why users consider on not using contact lens. As shown in Table 1 below, the inventor of the present application surprisingly found that, compared with the hydrogel lens without adding rotaxane compound (Comparative Example 1), the hydrogel lens added with rotaxane compound (Exemplary Examples 1 to 4) has a lower dehydration rate. In other words, the drying rate of the contact lens added with rotaxane compound is relatively slow, and the user is less likely to feel the rapid loss of moisture in the contact lens.

The measurement of elongation is used to evaluate a stretch elasticity of a lens body of a contact lens. The measurement method of elongation is using a ruler to measure the lens body after hydration and swelling. More specifically, the measurement method is fixing two ends of the lens body along its central axis at a relative distance of 0.25 cm (i.e., visible length of the lens body is 0.5 cm), in which a left fixed end of the lens body is aligned with the ruler; stretching the lens body toward a right side of the lens body along the direction of its central axis with a fixed force; and recording the final length (cm) of the lens body before the lens body is ruptured. The calculating of the elongation is: subtracting the length value before stretching from the length value after stretching, and then dividing by the length value before stretching to obtain an elongation value, which is expressed as a percentage.

The calculation formula of elongation is:

$$\text{Elongation (\%)} = \frac{\text{(length value after stretching - length value before stretching)}}{\text{length value before stretching}} * 100\%$$

The measurement of elongation is one of the important indicators for evaluating the property of a contact lens. If the contact lens is too hard (i.e., the elongation thereof is less than 150%), the contact lens will easily cause a user to feel uncomfortable when the user wears the contact lens on his eye. If the contact lens is too soft (i.e., the elongation thereof is greater than 500%), it would be difficult for users preparing to wear the contact lens to place the contact lens flatly on their fingertips. The hydrogel lenses made according to the following Exemplary Examples 1 to 4 have elongations between 250% and 380%, and more specifically between 320% and 380%.

The measurement of lubricity is one of the important indicators for evaluating the wearing comfort of a contact lens. The contact lens with good lubricity can provide a user with good wearing comfort. The evaluation method of the

lubricity is to use a blind test. The score of the blind test is 1 point to 10 points. The higher the score is, the better the lubricity is. According to the following Exemplary Examples 1 to 4, the hydrogel lenses all have a score of about 5 points.

The measurement of water content refers to the amount of water absorbed by a lens body of a contact lens under equilibrium. The water content is determined according to ISO18369-4. The water content can be measured by: taking the lens body after hydration and swelling; placing the lens body on a damp cloth and gently wiping the lens body to remove excess water; putting the lens body in a sample pan on a scale, and weighing and recording the weight (a) of the lens body before dehydration; and then putting the lens body in an oven with a constant temperature and a constant humidity for several hours, and weighing and recording the weight (b) of the lens body after dehydration. The water content is calculated by subtracting the weight (b) of the lens body after dehydration from the weight (a) of the lens body before dehydration, and then dividing by the weight (a) of the lens body before dehydration to obtain a calculated value. The calculated value is the water content of the lens body. The water content can be expressed as a percentage. The hydrogel lenses made according to the following Exemplary Examples 1 to 4 all have the water content between 10% and 75% (w/w), specifically between 32% and 70% (w/w), and more specifically between 36% and 65 %.

The calculation formula for water content is:

$$\text{Water Content (\%)} = \frac{\text{weight (a) of lens body before dehydration - weight (b) of lens body after dehydration}}{\text{weight (a) of lens body before dehydration}} * 100\%$$

It should be noted that, in the following Table 1, the base curve (BC) and the diameter (DIA) of the lens body are measured by a contact lens optical measuring instrument. The central thickness (CT) of the lens body is measured by a lens thickness detector. The refractive index of the lens body is measured by an Abbe refraction-meter. The light transmittance of the lens body is obtained by measuring the visible light transmittance value using an ultraviolet spectrometer.

[Comparative Example 1 and Exemplary Examples 1 to 4]

The following Table 1 lists the hydrogel compositions of Comparative Example 1 (control group) and Exemplary Examples 1 to 4, and Table 1 also shows the test results of the physical and chemical properties of the hydrogel lenses. The above Exemplary Examples are merely illustrative and are not intended to limit the scope of the present disclosure.

Specifically, the hydrogel compositions of Comparative Example 1 and Exemplary Examples 1 to 4 all include: 85.28 wt% of hydrophilic monomer, 0.88 wt% of cross-linker, 0.50 wt% of initiator, 1.11 wt% of UV blocking monomer, 12.21 wt% of co-solvent, and 0.02 wt% of dye. Among them, Comparative Example 1 is a control group, which does not contain rotaxane compound, while the Exemplary Examples 1 to 4 are added with different weight ratio of rotaxane compound. In the rotaxane compounds of Exemplary Examples 1 to 4, cyclodextrin is used as the cyclic molecule, and polyethylene glycol (PEG) is used as the linear molecule.

[Table 1]

| | Comparative Example 1 (control group) | Exemplary Example 1 | Exemplary Example 2 | Exemplary Example 3 | Exemplary Example 4 |
|---|---|---|---|---|---|
| rotaxane compound (wt%) | 0 | 4.00 | 5.10 | 6.90 | 1.35 |
| cyclic molecule: linear molecule (weight ratio) | 0 | 20.5:1 | 11.9:1 | 8.80:1 | 30.6:1 |
| base curve BC (mm) | 8.425 | 8.450 | 8.425 | 8.500 | 8.475 |
| central thickness CT (mm) | 0.082 | 0.078 | 0.090 | 0.086 | 0.085 |
| diameter DIA (mm) | 14.15 | 14.15 | 14.10 | 14.05 | 14.15 |
| refractive index | 1.4044 | 1.4054 | 1.4051 | 1.4044 | 1.4049 |

(continued)

| | Comparative Example 1 (control group) | Exemplary Example 1 | Exemplary Example 2 | Exemplary Example 3 | Exemplary Example 4 |
|---|---|---|---|---|---|
| light transmittance (%) | 98.707 | 99.027 | 98.797 | 98.931 | 99.125 |
| water content (%) | 57.21 | 56.82 | 57.23 | 56.82 | 57.10 |
| dynamic contact angle DCA (degree) | 30.45 | 26.49 | 25.95 | 29.33 | 25.66 |
| elongation (%) | 320 | 340 | 320 | 330 | 350 |
| atmospheric dehydration rate at $10^{th}$ minute (%) | 12.40 | 10.88 | 10.95 | 10.85 | 10.78 |
| atmospheric dehydration rate at $30^{th}$ minute (%) | 34.04 | 30.35 | 31.25 | 32.20 | 30.65 |
| atmospheric dehydration rate at $60^{th}$ minute (%) | 52.21 | 50.09 | 50.55 | 50.75 | 51.52 |
| lubricity (1~10 points) | 5.0 | 5.0 | 4.9 | 5.1 | 5.0 |
| water extraction rate (%) | 0.21 | 0.11 | 0.08 | 0.75 | 0.10 |
| hexane extraction rate (%) | 0.09 | 0.12 | 0.09 | 0.09 | 0.11 |

As shown in Table 1, compared to the hydrogel lens without rotaxane compound (Comparative Example 1, control group), the hydrogel lenses added with rotaxane compound (Exemplary Example 1 to Exemplary Example 4) have lower dynamic contact angles. In other words, the contact lenses added with rotaxane compound have better wettability.

As shown in Table 1, compared to the hydrogel lens without rotaxane compound (Comparative Example 1, control group), the hydrogel lenses added with rotaxane compound (Exemplary Example 1 to Exemplary Example 4) have lower atmospheric dehydration rates. In other words, the drying rates of the hydrogel lenses added with rotaxane compound are relatively slow, so that the user (wearer) is less likely to feel dryness from the lenses.

As shown in Table 1, the performances of the basic physical and chemical properties (i.e., the base curve, the center thickness, the diameter, the refractive index, the elongation, the transmittance, the lubricity) of the hydrogel lenses added with rotaxane compound (Exemplary Example 1 to Exemplary Example 4) are not inferior to that of the hydrogel lens without rotaxane compound (Comparative Example 1, control group). That is, since the rotaxane compound is introduced into the hydrogel lens, the hydrogel lens not only has better wettability, lower atmospheric dehydration rate, potential for loading and slowly releasing active ingredients, but also can maintain the characteristics for contact lens requirements.

Furthermore, the water extraction rate (%) and the hexane extraction rate (%) of the hydrogel lenses in Table 1 are relatively low, which shows that the curing of the hydrogel compositions is quite complete.

[Clinical Evaluation of Wearing Status of Contact Lens]

Two methods of clinical evaluation of wearing status of contact lens commonly used in practical operation are introduced as follows, which include questionnaire interviews and tear meniscus height.

Questionnaire interview (subjective test) is to use contact lens questionnaire to ask about the symptoms of wearing, the frequency of occurrence, and the feeling of wearing for a long time to understand the actual condition of the wearer.

The evaluation method of the questionnaire interview is to immerse the hydrogel lenses made of Comparative

Example 1 and Exemplary Examples 1 to 4 of Table 1 in a contact lens preservation solution containing menthol, thereby loading the menthol on the hydrogel lenses; and then take out the hydrogel lenses. Next, five subjects for a comprehensive evaluation test are selected. The test was completed by the five subjects aged between 25 and 40 years old. All the five subjects performed the test without knowing the composition ratio of the hydrogel lenses and the added ingredients of the drug. The score is on a scale from 1 to 10 points. The higher the score is, the stronger the feedback provided by the subjects of the test item is. The comprehensive evaluation test results of the five subjects are shown in Table 2.

[Table 2]

| evaluation item | wear time of contact lens | Comparative Example 1 | Exemplary Examples 1 to 4 |
|---|---|---|---|
| cooling sensation | just worn | 6 | 6.2~6.5 |
| | worn for 30 minutes | 2.8 | 3.8~4.4 |
| | worn for 1 hour | 1.2 | 2.0~2.5 |
| | worn for 2 hours | 0.6 | 1.1~1.7 |
| drying sensation | just worn | 2.2 | 1.8~2.0 |
| | worn for 4 hours | 3.2 | 2.0~2.2 |
| foreign body sensation | just worn | 1.8 | 1.5~1.7 |
| | worn for 4 hours | 3.4 | 1.7~2.0 |

Tear meniscus height is observed by using a corneal topography system, and the height of the tear river is measured by observing the tear layer to determine whether a subject’s tears are sufficient. The height of a normal person’s tear channel is about 0.25 mm. If the measured height is lower than 0.25 mm, the test result means that there is insufficient tear secretion.

The evaluation method of the tear meniscus height is to immerse the hydrogel lenses made of Comparative Example 1 and Exemplary Examples 1 to 4 of Table 1 in a contact lens preservation solution containing menthol, thereby loading the menthol on the hydrogel lenses; and then take out the hydrogel lenses. Next, five subjects were selected, and the tear meniscus height was measured to determine the amount of tear secretion of the subjects. When the menthol continues to remain active, the menthol stimulates the secretion of tears. The test was completed by the five subjects aged between 25 and 40 years old. All the five subjects performed the test without knowing the composition ratio of the hydrogel lenses and the added ingredients of the drug. The comprehensive evaluation test results of the five subjects are shown in Table 3.

[Table 3]

| evaluation item | wear time of contact lens | Comparative Example 1 | Exemplary Examples 1 to 4 |
|---|---|---|---|
| tear meniscus height (mm) | just worn | 0.300 | 0.310~0.325 |
| | worn for 2 hours | 0.240 | 0.282~0.298 |
| | worn for 4 hours | 0.230 | 0.265~0.272 |

[Beneficial Effects of the Embodiments]

In conclusion, by virtue of "introduction of a rotaxane compound into a hydrogel composition", the hydrogel composition and the hydrogel lens of the present disclosure can have effects of loading and slowly releasing active ingredients.

It is worth mentioning that, although the rotaxane compound is introduced into the hydrogel lens, the hydrogel lens of the present disclosure can not only have the effects of loading and slowly releasing the active ingredients, but also maintain required characteristics of the hydrogel lens, such as the lens base curve (BC), the lens center thickness (CT), the lens diameter (DIA), the refractive index, the visible light transmittance, and the dynamic contact angle.

## Claims

**1.** A hydrogel composition (100), **characterized by** comprising:

a hydrophilic monomer (2), a content range of the hydrophilic monomer (2) in the hydrogel composition (100) being between 60 wt% and 99.85 wt.% based on a total weight of the hydrogel composition (100);
a cross-linker, a content range of the cross-linker in the hydrogel composition (100) being between 0.01 wt% and 1 wt% based on the total weight of the hydrogel composition (100);
an initiator, a content range of the initiator in the hydrogel composition (100) being between 0.01 wt% and 2 wt% based on the total weight of the hydrogel composition (100); and
a rotaxane compound (1), a content range of the rotaxane compound (1) in the hydrogel composition (100) being between 0.1 wt% and 15 wt% based on the total weight of the hydrogel composition (100); wherein the rotaxane compound (1) includes at least one cyclic molecule (12) and at least one linear molecule (11) passing through the at least one cyclic molecule (12) in a string manner;
wherein a weight ratio of the rotaxane compound (1) relative to the hydrophilic monomer (2) is between 1:6 and 1:99;
wherein, in the rotaxane compound (1), a number average molecular weight of the linear molecule (11) is between 2,000 and 20,000, and the rotaxane compound (1) does not include any slopper or capping used for preventing the cyclic molecule (12) from detaching from two ends of the linear molecule (11).

**2.** The hydrogel composition (100) according to claim 1, wherein the content range of the rotaxane compound (1) in the hydrogel composition (100) is between 0.1 wt% and 12 wt%.

**3.** The hydrogel composition (100) according to claim 2, wherein the content range of the rotaxane compound (1) in the hydrogel composition (100) is between 0.5 wt% and 8 wt%.

**4.** The hydrogel composition (100) according to claim 1, wherein the weight ratio of the rotaxane compound (1) relative to the hydrophilic monomer (2) is between 1:11 and 1:99.

**5.** The hydrogel composition (100) according to claim 4, wherein the weight ratio of the rotaxane compound (1) relative to the hydrophilic monomer (2) is between 1:14 and 1:74.

**6.** The hydrogel composition (100) according to claim 1, wherein, in the rotaxane compound (1), a weight ratio of the at least one cyclic molecule (12) relative to the at least one linear molecule (11) is between 1:1 and 50:1.

**7.** The hydrogel composition (100) according to claim 6, wherein, in the rotaxane compound (1), the weight ratio of the at least one cyclic molecule (12) relative to the at least one linear molecule (11) is between 5:1 and 35:1.

**8.** The hydrogel composition (100) according to claim 1, wherein the at least one cyclic molecule (12) is cyclodextrin or a derivative thereof.

**9.** The hydrogel composition (100) according to claim 8, wherein the cyclodextrin is at least one material selected from a group consisting of α-cyclodextrin, β-cyclodextrin, γ-cyclodextrin, hydroxypropyl-β-cyclodextrin, (2-hydroxypropyl)-γ-cyclodextrin, sulfobutylether-β-cyclodextrin, and methyl-β-cyclodextrin.

**10.** The hydrogel composition (100) according to claim 1, wherein the at least one cyclic molecule (12) is crown ether or a derivative thereof.

**11.** The hydrogel composition (100) according to claim 10, wherein the crown ether is at least one material selected from a group consisting of 12-crown-4, 15-crown-5, 18-crown-6, benzo-18-crown-6, benzo-15-crown-5, dicyclohexyl-18-crown-6, 2-(hydroxymethyl)-12-crown-4-ether, 2-(hydroxymethyl)-15-crown-5-ether, and 2-(hydroxymethyl)-18-crown-6-ether.

**12.** The hydrogel composition (100) according to claim 1, wherein the at least one linear molecule (11) is at least one material selected from a group consisting of polyethylene glycol (PEG), polyvinyl alcohol (PVA), and polypropylene glycol (PPG).

**13.** The hydrogel composition (100) according to claim 12, wherein

the polyethylene glycol has a chemical structure of formula (1):

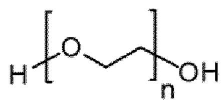 (1);

the polyvinyl alcohol has a chemical structure of formula (2):

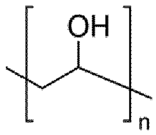 (2);

and
the polypropylene glycol has a chemical structure of formula (3):

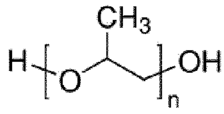 (3);

wherein "n" is a positive integer greater than 4.

**14.** The hydrogel composition (100) according to claim 1, wherein the content range of the hydrophilic monomer (2) in the hydrogel composition (100) is between 68.88 wt% and 99.85 wt%.

**15.** The hydrogel composition (100) according to claim 1, wherein the hydrophilic monomer (2) is at least one material selected from a group consisting of N-vinyl pyrrolidone (NVP), 2-hydroxyethyl methacrylate (HEMA), methacrylic acid (MAA), methyl methacrylate (MMA), acrylic acid (AAc), N,N-dimethyl acrylamide (DMA), 2,3-dihydroxypropyl methacrylate (GMMA), N,N-dimethyl methacrylamide, and N-vinyl-N-methyl acetamide.

**16.** The hydrogel composition (100) according to claim 1, wherein the hydrophilic monomer (2) has a vinyl group, an acetyl group, a propenyl group or an acryl group.

**17.** The hydrogel composition (100) according to claim 1, wherein the content range of the cross-linker in the hydrogel composition (100) is between 0.04 wt% and 0.57 wt%.

**18.** The hydrogel composition (100) according to claim 1, wherein the cross-linker is at least one material selected from a group consisting of ethylene glycol dimethacrylate, diethylene glycol dimethacrylate, triethylene glycol dimethacrylate, tetraethylene glycol dimethacrylate, allyl methacrylate, triethylene glycol dially ether, tetraethylene glycol dially ether, and 1,1,1-trimethylolpropane trimethacrylate.

**19.** The hydrogel composition (100) according to claim 1, wherein the initiator is a photo-initiator, and a content range of the photo-initiator in the hydrogel composition (100) is between 0.05 wt% and 0.72 wt%.

**20.** The hydrogel composition (100) according to claim 19, wherein the photo-initiator is at least one material selected from a group consisting of bis(2,6-difluoro-3-(1-hydropyrro-1-yl)- phenyl)titanocene, phenylbis-(2,4,6-trimethylbenzoyl)-phosphine oxide, and 2-hydroxy-2-methyl-1-phenyl-1-porpanone.

**21.** The hydrogel composition (100) according to claim 1, further comprising an ultraviolet light blocking monomer, and a content range of the ultraviolet light blocking monomer in the hydrogel composition (100) being between 0.34 wt% and 1.68 wt%.

**22.** The hydrogel composition (100) according to claim 21, wherein the ultraviolet light blocking monomer is at least one material selected from a group consisting of benzophenone and benzotriazole.

**23.** The hydrogel composition (100) according to claim 1, further comprising a co-solvent, and a content range of the co-solvent in the hydrogel composition (100) being between 4.33 wt% and 12.67 wt%.

**24.** The hydrogel composition (100) according to claim 23, wherein the co-solvent is at least one material selected from a group consisting of glycerol, isopropyl alcohol, n-butanol, t-butanol, t-amyl alcohol, and n-hexanol.

**25.** The hydrogel composition (100) according to claim 1, further comprising a dye, and a content range of the dye in the hydrogel composition (100) being between 0.002 wt% and 0.039 wt%.

**26.** The hydrogel composition (100) according to claim 25, wherein the dye is at least one material selected from a group consisting of reactive blue 19 (disodium 1-amino-9,10-dioxo-4-[3-(2-sulfonatooxyethylsulfonyl) anilino] anthracene-2-sulfonate), Sudan III (1-[4-(phenylazo)phenylazo]-2-naphthol, Indigo (2,2'-bis(2,3-dihydro- 3-oxoindolylidene)), and Quinoline Yellow (disodium 2-(1,3-dioxo- 2,3-dihydro-1H-inden-2-yl) quinolone-6,8-disulfonate).

**27.** The hydrogel composition (100) according to claim 1, wherein the hydrophilic monomer (2) has an acryl group, and the weight ratio of the rotaxane compound (1) relative to the hydrophilic monomer (2) is between 1:6 and 1:94.

**28.** The hydrogel composition (100) according to claim 27, wherein, when the cyclic molecule (12) in the rotaxane compound (1) is α-cyclodextrin or β-cyclodextrin, the weight ratio of the rotaxane compound (1) relative to the hydrophilic monomer (2) is between 1:11 and 1:54; wherein, when the cyclic molecule (12) in the rotaxane compound (1) is hydroxypropyl-β-cyclodextrin, the weight ratio of the rotaxane compound (1) relative to the hydrophilic monomer (2) is between 1:17 and 1:86; wherein, when the cyclic molecule (12) in the rotaxane compound (1) is (2-hydroxypropyl)-γ-cyclodextrin, the weight ratio of the rotaxane compound (1) relative to the hydrophilic monomer (2) is between 1:19 and 1:94.

**29.** The hydrogel composition (100) according to claim 1, wherein the number average molecular weight of the linear molecule (11) is not less than 4,000.

**30.** The hydrogel composition (100) according to claim 1, wherein, in the rotaxane compound (1), an outer periphery of the cyclic molecule (12) has a hydrophilic functional group, and an inner side of the cyclic molecule (12) has a hydrophobic cavity structure; wherein the rotaxane compound (1) interacts with the hydrophilic monomer (2) through the hydrophilic functional group on the outer periphery of the cyclic molecule (12), so that the rotaxane compound (1) is dispersed in the hydrogel composition (100).

**31.** The hydrogel composition (100) according to claim 1, wherein the linear molecule (11) of the rotaxane compound (1) is dispersed in the hydrophilic monomer (2) in a tortuous form, the rotaxane compound (1) further includes a plurality of cyclic molecules (12), and the plurality of cyclic molecules (12) are threaded onto the linear molecule (11) in a series connection.

**32.** The hydrogel composition (100) according to claim 31, wherein the plurality of series-connected cyclic molecules (12) and adjacent ones of the plurality of series-connected cyclic molecules (12) are capable of being attracted to each other by a hydrogen bonding force and are arranged in a stacked manner.

**33.** The hydrogel composition (100) according to claim 31, wherein, on the linear molecule (11), the plurality of series-connected cyclic molecules (12) are arranged in a heads to heads and tails to tails manner.

**34.** A hydrogel lens, **characterized by** comprising: a lens body that is formed by the hydrogel composition (100) as claimed in claim 1.

**35.** The hydrogel lens according to claim 34, wherein the rotaxane compound (1) is distributed on at least one surface of a concave arc surface or a convex arc surface of the lens body to form a rotaxane layer, and the rotaxane compound (1) is configured to carry an active ingredient.

**36.** The hydrogel lens according to claim 35, wherein the active ingredient is at least one material selected from a group consisting of menthol, d-camphor, d-borneol, potassium chloride, lysozyme hydrochloride, dipotassium glycyrrhizinate, aminocaproic acid, ε-aminocaproic acid, 6-aminohexanoic acid, zinc sulfate, vitamin B2, sodium azulene sulfonic acid, sulfamethoxazole, prano-profen, naphazoline, tetrahydrozoline, chlorobutanol, phenylephrine hydrochloride, phenylephrine, vitamin E, L-potassium aspartate, magnesium L-aspartate, chondroitin sulfate, neostigmine methylsulfate, vitamin B12, vitamin B5, vitamin B6, vitamin A, taurine, diphenhydramine, chlorpheniraminem, tranilast, boric acid, glycerine, hyaluronic acid, polyol, and allantoin.

**37.** The hydrogel lens according to claim 34, wherein a refractive index of the lens body is between 1.315 and 1.598, a light transmittance of the lens body is not less than 90%, a water content of the lens body is between 10 w/w% and 75 w/w%, a dynamic contact angle of the lens body is not greater than 80 degrees, and an elongation of the lens body is between 250% and 380%.

## Patentansprüche

**1.** Hydrogelzusammensetzung (100), welche **dadurch gekennzeichnet ist, dass** sie Folgendes aufweist:

ein hydrophiles Monomer (2), wobei ein Gehaltsbereich des hydrophilen Monomers (2) in der Hydrogelzusammensetzung (100) zwischen 60 Gew.-% und 99,85 Gew.-%, bezogen auf ein Gesamtgewicht der Hydrogelzusammensetzung (100), ist;
einen Vernetzer, wobei ein Gehaltsbereich des Vernetzers in der Hydrogelzusammensetzung (100) zwischen 0,01 Gew.-% und 1 Gew.-%, bezogen auf das Gesamtgewicht der Hydrogelzusammensetzung (100), ist;
einen Initiator, wobei ein Gehaltsbereich des Initiators in der Hydrogelzusammensetzung (100) zwischen 0,01 Gew.-% und 2 Gew.-% bezogen auf das Gesamtgewicht der Hydrogelzusammensetzung (100) ist; und
eine Rotaxanverbindung (1), wobei ein Gehaltsbereich der Rotaxanverbindung (1) in der Hydrogelzusammensetzung (100) zwischen 0,1 Gew.-% und 15 Gew.-%, bezogen auf das Gesamtgewicht der Hydrogelzusammensetzung (100), ist; wobei die Rotaxanverbindung (1) mindestens ein zyklisches Molekül (12) und mindestens ein lineares Molekül (11) aufweist, welches durch das mindestens eine zyklische Molekül (12) fadenförmig hindurchgeht;
wobei ein Gewichtsverhältnis der Rotaxanverbindung (1) relativ zum hydrophilen Monomer (2) zwischen 1:6 und 1:99 ist;
wobei, in der Rotaxanverbindung (1), ein Zahlenmittel des Molekulargewichts des linearen Moleküls (11) zwischen 2 000 und 20 000 ist, und die Rotaxanverbindung (1) weder einen Slopper noch ein Capping zum Verhindern, dass das zyklische Molekül (12) sich von zwei Enden des linearen Moleküls (11) ablöst, aufweist.

**2.** Hydrogelzusammensetzung (100) gemäß Anspruch 1, wobei der Gehaltsbereich der Rotaxanverbindung (1) in der Hydrogelzusammensetzung (100) zwischen 0,1 Gew.-% und 12 Gew.-% ist.

**3.** Hydrogelzusammensetzung (100) gemäß Anspruch 2, wobei der Gehaltsbereich der Rotaxanverbindung (1) in der Hydrogelzusammensetzung (100) zwischen 0,5 Gew.-% und 8 Gew.-% ist.

**4.** Hydrogelzusammensetzung (100) gemäß Anspruch 1, wobei das Gewichtsverhältnis der Rotaxanverbindung (1) relativ zum hydrophilen Monomer (2) zwischen 1:11 und 1:99 ist.

**5.** Hydrogelzusammensetzung (100) gemäß Anspruch 4, wobei das Gewichtsverhältnis der Rotaxanverbindung (1) relativ zum hydrophilen Monomer (2) zwischen 1:14 und 1:74 ist.

**6.** Hydrogelzusammensetzung (100) gemäß Anspruch 1, wobei, in der Rotaxanverbindung (1), ein Gewichtsverhältnis des mindestens einen zyklischen Moleküls (12) relativ zum mindestens einen linearen Molekül (11) zwischen 1:1 und 50:1 ist.

**7.** Hydrogelzusammensetzung (100) gemäß Anspruch 6, wobei, in der Rotaxanverbindung (1), das Gewichtsverhältnis des mindestens einen zyklischen Moleküls (12) relativ zum mindestens einen linearen Molekül (11) zwischen 5:1 und 35:1 ist.

**8.** Hydrogelzusammensetzung (100) gemäß Anspruch 1, wobei das zumindest eine zyklische Molekül (12) Cyclodextrin oder ein Derivat davon ist.

**9.** Hydrogelzusammensetzung (100) gemäß Anspruch 8, wobei das Cyclodextrin mindestens ein Material ist, welches aus einer Gruppe ausgewählt ist, die aus α-Cyclodextrin, β-Cyclodextrin, γ-Cyclodextrin, Hydroxypropyl-β-cyclodextrin, (2-Hydroxypropyl)-γ-cyclodextrin, Sulfobutylether-β-cyclodextrin und Methyl-β-cyclodextrin besteht.

**10.** Hydrogelzusammensetzung (100) gemäß Anspruch 1, wobei das mindestens eine zyklische Molekül (12) Kronenether oder ein Derivat davon ist.

**11.** Hydrogelzusammensetzung (100) gemäß Anspruch 10, wobei der Kronenether mindestens ein Material ist, das aus einer Gruppe ausgewählt ist, die aus 12-Krone-4-, 15-Krone-5-, 18-Krone-6-, Benzo-18-Krone-6-, Benzo-15-Krone-5, Dicyclohexyl-18-Krone-6, 2-(Hydroxymethyl)-12-Krone-4-Ether, 2-(Hydroxymethyl)-15-Krone-5-Ether und 2-(Hydroxymethyl)-18-Krone-6-Ether besteht.

**12.** Hydrogelzusammensetzung (100) gemäß Anspruch 1, wobei das mindestens eine lineare Molekül (11) mindestens ein Material ist, das aus einer Gruppe ausgewählt ist, die aus Polyethylenglykol (PEG), Polyvinylalkohol (PVA) und Polypropylenglykol (PPG) besteht.

**13.** Hydrogelzusammensetzung (100) gemäß Anspruch 12, wobei

das Polyethylenglykol eine chemische Struktur der Formel (1) hat:

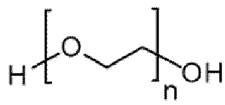

(1);

der Polyvinylalkohol eine chemische Struktur der Formel (2) hat:

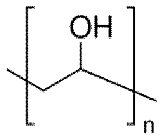

(2);

und
das Polypropylenglykol eine chemische Struktur der Formel (3) hat:

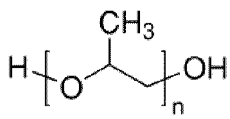

(3);

wobei "n" eine positive ganze Zahl größer als 4 ist.

**14.** Hydrogelzusammensetzung (100) gemäß Anspruch 1, wobei der Gehaltsbereich des hydrophilen Monomers (2) in der Hydrogelzusammensetzung (100) zwischen 68,88 Gew.-% und 99,85 Gew.-% ist.

**15.** Hydrogelzusammensetzung (100) gemäß Anspruch 1, wobei das hydrophile Monomer (2) mindestens ein Material ist, das aus einer Gruppe ausgewählt ist, die aus N-Vinylpyrrolidon (NVP), 2-Hydroxyethylmethacrylat (HEMA), Methacrylsäure (MAA), Methylmethacrylat (MMA), Acrylsäure (AAc), N,N-Dimethylacrylamid (DMA), 2,3-Dihydroxypropylmethacrylat (GMMA), N,N-Dimethylmethacrylamid und N-Vinyl-N-methylacetamid.

**16.** Hydrogelzusammensetzung (100) gemäß Anspruch 1, wobei das hydrophile Monomer (2) eine Vinylgruppe, eine Acetylgruppe, eine Propenylgruppe oder eine Acrylgruppe hat.

**17.** Hydrogelzusammensetzung (100) gemäß Anspruch 1, wobei der Gehaltsbereich des Vernetzers in der Hydrogelzusammensetzung (100) zwischen 0,04 Gew.-% und 0,57 Gew.-% ist.

**18.** Hydrogelzusammensetzung (100) gemäß Anspruch 1, wobei der Vernetzer mindestens ein Material ist, das aus einer Gruppe ausgewählt ist, die aus Ethylenglykoldimethacrylat, Diethylenglykoldimethacrylat, Triethylenglykoldimethacrylat, Tetraethylenglykoldimethacrylat, Allylmethacrylat, Triethylenglykoldiallylether, Tetraethylenglykoldiallylether und 1,1,1-Trimethylolpropantrimethacrylat besteht.

**19.** Hydrogelzusammensetzung (100) gemäß Anspruch 1, wobei der Initiator ein Photoinitiator ist, und ein Gehaltsbereich des Photoinitiators in der Hydrogelzusammensetzung (100) zwischen 0,05 Gew.-% und 0,72 Gew.-% ist.

**20.** Hydrogelzusammensetzung (100) gemäß Anspruch 19, wobei der Photoinitiator mindestens ein Material ist, das aus einer Gruppe ausgewählt ist, die aus Bis(2,6-difluoro-3-(1-hydropyrro-1-yl)-phenyl)titanocen, Phenylbis-(2,4,6-trimethylbenzoyl)-phosphinoxid und 2-Hydroxy-2-methyl-1-phenyl-1-porpanon besteht.

**21.** Hydrogelzusammensetzung (100) gemäß Anspruch 1, welche ferner ein ultraviolettes Licht blockierendes Monomer aufweist, wobei ein Gehaltsbereich des ultraviolettes Licht blockierenden Monomers in der Hydrogelzusammensetzung (100) zwischen 0,34 Gew.-% und 1,68 Gew.-% ist.

**22.** Hydrogelzusammensetzung (100) gemäß Anspruch 21, wobei das ultraviolettes Licht blockierende Monomer mindestens ein Material ist, das aus einer Gruppe ausgewählt ist, die aus Benzophenon und Benzotriazol besteht.

**23.** Hydrogelzusammensetzung (100) gemäß Anspruch 1, welche ferner ein Co-Lösungsmittel aufweist, wobei ein Gehaltsbereich des Co-Lösungsmittels in der Hydrogelzusammensetzung (100) zwischen 4,33 Gew.-% und 12,67 Gew.-% ist.

**24.** Hydrogelzusammensetzung (100) gemäß Anspruch 23, wobei das Co-Lösungsmittel mindestens ein Material ist, das aus einer Gruppe ausgewählt ist, die aus Glycerin, Isopropylalkohol, n-Butanol, t-Butanol, t-Amylalkohol und n-Hexanol besteht.

**25.** Hydrogelzusammensetzung (100) gemäß Anspruch 1, welche ferner einen Farbstoff aufweist, und ein Gehaltsbereich des Farbstoffs in der Hydrogelzusammensetzung (100) zwischen 0,002 Gew.-% und 0,039 Gew.-% ist.

**26.** Hydrogelzusammensetzung (100) gemäß Anspruch 25, wobei der Farbstoff mindestens ein Material ist, das aus einer Gruppe ausgewählt ist, die aus Reactive Blue 19 (Dinatrium-1-amino-9,10-dioxo-4-[3-(2-sulfonatooxyethylsulfonyl) anilino]anthracen-2-sulfonat), Sudan III (1-[4-(Phenylazo)phenylazo]-2-naphthol, Indigo (2,2'-Bis(2,3-dihydro-3-oxoindolyliden)) und Chinolingelb (Dinatrium-2-(1,3-dioxo-2,3-dihydro-1H-inden-2-yl)chinolin-6,8-disulfonat) besteht.

**27.** Hydrogelzusammensetzung (100) gemäß Anspruch 1, wobei das hydrophile Monomer (2) eine Acrylgruppe hat, und das Gewichtsverhältnis der Rotaxanverbindung (1) relativ zum hydrophilen Monomer (2) zwischen 1:6 und 1:94 ist.

**28.** Hydrogelzusammensetzung (100) gemäß Anspruch 27, wobei, wenn das zyklische Molekül (12) in der Rotaxanverbindung (1) $\alpha$-Cyclodextrin oder $\beta$-Cyclodextrin ist, das Gewichtsverhältnis der Rotaxanverbindung (1) relativ zum hydrophilen Monomer (2) zwischen 1:11 und 1:54 ist; wobei, wenn das zyklische Molekül (12) in der Rotaxanverbindung (1) Hydroxypropyl-$\beta$-cyclodextrin ist, das Gewichtsverhältnis der Rotaxanverbindung (1) relativ zum hydrophilen Monomer (2) zwischen 1:17 und 1:86 ist; wobei, wenn das zyklische Molekül (12) in der Rotaxanverbindung (1) (2-Hydroxypropyl)-$\gamma$-cyclodextrin ist, das Gewichtsverhältnis der Rotaxanverbindung (1) relativ zum hydrophilen Monomer (2) zwischen 1:19 und 1:94 ist.

**29.** Hydrogelzusammensetzung (100) gemäß Anspruch 1, wobei das Zahlenmittel des Molekulargewichts des linearen Moleküls (11) nicht weniger als 4 000 ist.

**30.** Hydrogelzusammensetzung (100) gemäß Anspruch 1, wobei, in der Rotaxanverbindung (1), eine äußere Umfangsfläche des zyklischen Moleküls (12) eine hydrophile funktionelle Gruppe hat, und eine Innenseite des zyklischen Moleküls (12) eine hydrophobe Hohlraumstruktur hat; wobei die Rotaxanverbindung (1) mit dem hydrophilen Monomer (2) durch die hydrophile funktionelle Gruppe an der äußeren Umfangsfläche des zyklischen Moleküls (12) wechselwirkt, sodass die Rotaxanverbindung (1) in der Hydrogelzusammensetzung (100) dispergiert ist.

**31.** Hydrogelzusammensetzung (100) gemäß Anspruch 1, wobei das lineare Molekül (11) der Rotaxanverbindung (1) in dem hydrophilen Monomer (2) in einer gewundenen Form dispergiert ist, die Rotaxanverbindung (1) ferner mehrere zyklische Moleküle (12) aufweist, und die mehreren zyklischen Moleküle (12) auf das lineare Molekül (11) in einer Reihenschaltung durchgefädelt sind.

**32.** Hydrogelzusammensetzung (100) gemäß Anspruch 31, wobei die mehreren in Reihe geschalteten zyklischen Moleküle (12) und benachbarte Moleküle der mehreren in Reihe geschalteten zyklischen Moleküle (12) in der Lage sind, durch eine Wasserstoffbindungskraft zueinander hingezogen zu sein und in einer gestapelten Weise angeordnet sind.

**33.** Hydrogelzusammensetzung (100) gemäß Anspruch 31, wobei, auf dem linearen Molekül (11), die mehreren in Reihe geschalteten zyklischen Moleküle (12) in einer Kopf-zu-Kopf- und Schwanz-zu-Schwanz-Weise angeordnet sind.

**34.** Hydrogellinse, **dadurch gekennzeichnet, dass** sie aufweist: einen Linsenkörper, welcher mittels der Hydrogelzusammensetzung (100) gemäß Anspruch 1 gebildet ist.

**35.** Hydrogellinse gemäß Anspruch 34, wobei die Rotaxanverbindung (1) auf mindestens einer Oberfläche einer konkaven Bogenfläche oder einer konvexen Bogenfläche des Linsenkörpers verteilt ist, um eine Rotaxanschicht zu bilden, und die Rotaxanverbindung (1) konfiguriert ist, um einen Wirkstoff zu tragen.

**36.** Hydrogellinse gemäß Anspruch 35, wobei der Wirkstoff mindestens ein Material ist, das aus einer Gruppe ausgewählt ist, die aus Menthol, d-Campher, d-Borneol, Kaliumchlorid, Lysozymhydrochlorid, Dikaliumglycyrrhizinat, Aminocapronsäure, ε-Aminocapronsäure, 6-Aminohexansäure, Zinksulfat, Vitamin B2, Natriumazulensulfonsäure, Sulfamethoxazol, Pranoprofen, Naphazolin, Tetrahydrozolin, Chlorbutanol, Phenylephrinhydrochlorid, Phenylephrin, Vitamin E, L- Kaliumaspartat, Magnesium-L-aspartat, Chondroitinsulfat, Neostigminmethylsulfat, Vitamin B12, Vitamin B5, Vitamin B6, Vitamin A, Taurin, Diphenhydramin, Chlorpheniramin, Tranilast, Borsäure, Glycerin, Hyaluronsäure, Polyol und Allantoin besteht.

**37.** Hydrogellinse gemäß Anspruch 34, wobei ein Brechungsindex des Linsenkörpers zwischen 1,315 und 1,598 ist, eine Lichtdurchlässigkeit des Linsenkörpers nicht weniger als 90% ist, ein Wassergehalt des Linsenkörpers zwischen 10 w/w% und 75 w/w% ist, ein dynamischer Kontaktwinkel des Linsenkörpers nicht größer als 80 Grad ist und eine Elongation des Linsenkörpers zwischen 250% und 380% ist.

## Revendications

**1.** Composition d'hydrogel (100), **caractérisée par le fait qu'**elle comprend :

un monomère hydrophile (2), une plage de teneurs du monomère hydrophile (2) dans la composition d'hydrogel (100) étant comprise entre 60 % en poids et 99,85 % en poids sur la base d'un poids total de la composition d'hydrogel (100) ;
un agent de réticulation, une plage de teneurs de l'agent de réticulation dans la composition d'hydrogel (100) étant comprise entre 0,01 % en poids et 1 % en poids sur la base du poids total de la composition d'hydrogel (100) ;
un initiateur, une plage de teneurs de l'initiateur dans la composition d'hydrogel (100) étant comprise entre 0,01 % en poids et 2 % en poids sur la base du poids total de la composition d'hydrogel (100) ; et
un composé de rotaxane (1), une plage de teneurs du composé de rotaxane (1) dans la composition d'hydrogel (100) étant comprise entre 0,1 % en poids et 15 % en poids sur la base du poids total de la composition d'hydrogel (100) ; le composé de rotaxane (1) comprenant au moins une molécule cyclique (12) et au moins une molécule linéaire (11) passant à travers l'au moins une molécule cyclique (12) à la manière d'une corde ;
un rapport en poids du composé de rotaxane (1) par rapport au monomère hydrophile (2) étant compris entre 1 : 6 et 1 : 99 ;
dans le composé de rotaxane (1), un poids moléculaire moyen en nombre de la molécule linéaire (11) étant compris entre 2 000 et 20 000, et le composé de rotaxane (1) ne comprenant aucun bouchon ou aucune coiffe utilisé(e) pour empêcher que la molécule cyclique (12) ne se détache des deux extrémités de la molécule linéaire (11).

**2.** Composition d'hydrogel (100) selon la revendication 1, la plage de teneurs du composé de rotaxane (1) dans la composition d'hydrogel (100) étant comprise entre 0,1 % en poids et 12 % en poids.

**3.** Composition d'hydrogel (100) selon la revendication 2, la plage de teneurs du composé de rotaxane (1) dans la composition d'hydrogel (100) étant comprise entre 0,5 % en poids et 8 % en poids.

**4.** Composition d'hydrogel (100) selon la revendication 1, le rapport en poids du composé de rotaxane (1) par rapport au monomère hydrophile (2) étant compris entre 1 : 11 et 1 : 99.

**5.** Composition d'hydrogel (100) selon la revendication 4, le rapport en poids du composé de rotaxane (1) par rapport au monomère hydrophile (2) étant compris entre 1 : 14 et 1 : 74.

**6.** Composition d'hydrogel (100) selon la revendication 1, dans le composé de rotaxane (1), un rapport en poids de l'au moins une molécule cyclique (12) par rapport à l'au moins une molécule linéaire (11) étant compris entre 1 : 1 et 50 : 1.

**7.** Composition d'hydrogel (100) selon la revendication 6, dans le composé de rotaxane (1), le rapport en poids de l'au moins une molécule cyclique (12) par rapport à l'au moins une molécule linéaire (11) étant compris entre 5 : 1 et 35 : 1.

**8.** Composition d'hydrogel (100) selon la revendication 1, l'au moins une molécule cyclique (12) étant une cyclodextrine ou un dérivé correspondant.

**9.** Composition d'hydrogel (100) selon la revendication 8, la cyclodextrine étant au moins une matière choisie dans un groupe constitué par une $\alpha$-cyclodextrine, une $\beta$-cyclodextrine, une $\gamma$-cyclodextrine, une hydroxypropyl-$\beta$-cyclodextrine, une (2-hydroxypropyl-$\gamma$-cyclodextrine, une sulfobutyléther-$\beta$-cyclodextrine, et une méthyl-$\beta$-cyclodextrine.

**10.** Composition d'hydrogel (100) selon la revendication 1, l'au moins une molécule cyclique (12) étant un éther couronne ou un dérivé correspondant.

**11.** Composition d'hydrogel (100) selon la revendication 10, l'éther couronne étant au moins une matière choisie dans un groupe constitué par le 12-couronne-4, le 15-couronne-5, le 18-couronne-6, le benzo-18-couronne-6, benzo-15-couronne-5, le dicyclohexyl-18-couronne-6, le 2-(hydroxyméthyl)-12-couronne-4-éther, le 2-(hydroxyméthyl)-15-couronne-5-éther et le 2-(hydroxyméthyl)-18-couronne-6-éther.

**12.** Composition d'hydrogel (100) selon la revendication 1, l'au moins une molécule linéaire (11) étant au moins une matière choisie dans un groupe constitué par un polyéthylène glycol (PEG), un poly(alcool vinylique) (PVA) et un polypropylène glycol (PPG).

**13.** Composition d'hydrogel (100) selon la revendication 12,

le polyéthylène glycol ayant une structure chimique de formule (1) :

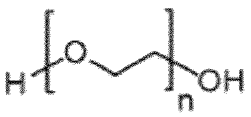

(1);

le poly(alcool vinylique) ayant une structure chimique de formule (2) :

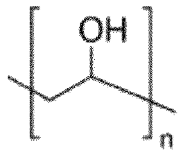

(2);

et
le polypropylène glycol ayant une structure chimique de formule (3) :

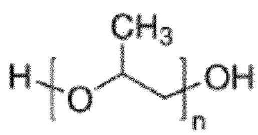

(3);

« n » étant un entier positif supérieur à 4.

**14.** Composition d'hydrogel (100) selon la revendication 1, la plage de teneurs du monomère hydrophile (2) dans la composition d'hydrogel (100) étant comprise entre 68,88 % en poids et 99,85 % en poids.

**15.** Composition d'hydrogel (100) selon la revendication 1, le monomère hydrophile (2) étant au moins une matière choisie dans un groupe constitué par la N-vinylpyrrolidone (NVP), le méthacrylate de 2-hydroxyéthyle (HEMA), l'acide méthacrylique (MAA), le méthacrylate de méthyle (MMA), l'acide acrylique (AAc), le N,N-diméthylacrylamide (DMA), le méthacrylate de 2,3-dihydroxypropyle (GMMA), le N,N-diméthylméthacrylamide et le N-vinyl-N-méthylacétamide.

**16.** Composition d'hydrogel (100) selon la revendication 1, le monomère hydrophile (2) ayant un groupe vinyle, un groupe acétyle, un groupe propényle ou un groupe acryle.

**17.** Composition d'hydrogel (100) selon la revendication 1, la plage de teneurs de l'agent de réticulation dans la composition d'hydrogel (100) étant comprise entre 0,04 % en poids et 0,57 % en poids.

**18.** Composition d'hydrogel (100) selon la revendication 1, l'agent de réticulation étant au moins une matière choisie dans un groupe constitué par le diméthacrylate d'éthylèneglycol, le diméthacrylate de diéthylèneglycol, le diméthacrylate de triéthylèneglycol, le diméthacrylate de tétraéthylèneglycol, le (méth)acrylate d'allyle, l'éther de diallyle de triéthylèneglycol, l'éther de diallyle de tétraéthylèneglycol et le triméthacrylate de 1,1,1-triméthylolpropane.

**19.** Composition d'hydrogel (100) selon la revendication 1, l'initiateur étant un photoinitiateur, et une plage de teneurs du photoinitiateur dans la composition d'hydrogel (100) étant comprise entre 0,05 % en poids et 0,72 % en poids.

**20.** Composition d'hydrogel (100) selon la revendication 19, le photoinitiateur étant au moins une matière choisie dans un groupe constitué par le bis(2,6-difluoro-3-(1-hydropyrro-1-yl)-phényl)titanocène, l'oxyde de phénylbis-(2,4,6-triméthylbenzoyl)-phosphine et la 2-hydroxy-2-méthyl-1-phényl-1-propanone.

**21.** Composition d'hydrogel (100) selon la revendication 1, comprenant en outre un monomère bloquant de la lumière ultraviolette, et une plage de teneurs du monomère bloquant de la lumière ultraviolette dans la composition d'hydrogel (100) étant comprise entre 0,34 % en poids et 1,68 % en poids.

**22.** Composition d'hydrogel (100) selon la revendication 21, le monomère bloquant de la lumière ultraviolette étant au moins une matière choisie dans un groupe constitué par une benzophénone et un benzotriazole.

**23.** Composition d'hydrogel (100) selon la revendication 1, comprenant en outre un cosolvant, et une plage de teneurs du cosolvant dans la composition d'hydrogel (100) étant comprise entre 4,33 % en poids et 12,67 % en poids.

**24.** Composition d'hydrogel (100) selon la revendication 23, le cosolvant étant au moins une matière choisie dans un groupe constitué par le glycérol, l'alcool isopropylique, le n-butanol, le t-butanol, l'alcool t-amylique et le n-hexanol.

**25.** Composition d'hydrogel (100) selon la revendication 1, comprenant en outre un colorant, et une plage de teneurs du colorant dans la composition d'hydrogel (100) étant comprise entre 0,002 % en poids et 0,039 % en poids.

**26.** Composition d'hydrogel (100) selon la revendication 25, le colorant étant au moins une matière choisie dans un groupe constitué par le bleu réactif 19 (1-amino-9,10-dioxo-4-[3-(2-sulfonatooxyéthylsulfonyl)anilino]anthracène-2-sulfonate disodique), le Soudan III (1-[4-(phénylazo)phénylazo]-2-naphtol, l'Indigo (2,2'-bis(2,3-dihydro-3-oxoindolylidène)) et le Jaune de quinoléine (2-(1,3-dioxo-2,3-dihydro-1H-indén-2-yl)quinolone-6,8-disulfonate disodique) .

**27.** Composition d'hydrogel (100) selon la revendication 1, le monomère hydrophile (2) ayant un groupe acryle, et le rapport en poids du composé de rotaxane (1) par rapport au monomère hydrophile (2) étant compris entre 1 : 6 et 1 : 94.

**28.** Composition d'hydrogel (100) selon la revendication 27, lorsque la molécule cyclique (12) dans le composé de rotaxane (1) est une α-cyclodextrine ou une β-cyclodextrine, le rapport en poids du composé de rotaxane (1) par rapport au monomère hydrophile (2) étant compris entre 1 : 11 et 1 : 54 ; lorsque la molécule cyclique (12) dans le composé de rotaxane (1) est une hydroxypropyl-β-cyclodextrine, le rapport en poids du composé de rotaxane (1) par rapport au monomère hydrophile (2) étant compris entre 1 : 17 et 1 : 86 ; lorsque la molécule cyclique (12) dans le composé de rotaxane (1) est une (2-hydroxypropyl)-γ-cyclodextrine, le rapport en poids du composé de rotaxane (1) par rapport au monomère hydrophile (2) étant compris entre 1 : 19 et 1 : 94.

**29.** Composition d'hydrogel (100) selon la revendication 1, le poids moléculaire moyen en nombre de la molécule linéaire

(11) n'étant pas inférieur à 4 000.

**30.** Composition d'hydrogel (100) selon la revendication 1, dans le composé de rotaxane (1), une périphérie extérieure de la molécule cyclique (12) ayant un groupe fonctionnel hydrophile, et un côté intérieur de la molécule cyclique (12) ayant une structure de cavité hydrophobe ; le composé de rotaxane (1) interagissant avec le monomère hydrophile (2) par le biais du groupe fonctionnel hydrophile sur la périphérie extérieure de la molécule cyclique (12), de sorte que le composé de rotaxane (1) soit dispersé dans la composition d'hydrogel (100) .

**31.** Composition d'hydrogel (100) selon la revendication 1, la molécule linéaire (11) du composé de rotaxane (1) étant dispersée dans le monomère hydrophile (2) sous une forme tortueuse, le composé de rotaxane (1) comprenant en outre une pluralité de molécules cycliques (12), et la pluralité de molécules cycliques (12) étant enfilées sur la molécule linéaire (11) dans une connexion en série.

**32.** Composition d'hydrogel (100) selon la revendication 31, la pluralité de molécules cycliques connectées en série (12) et des molécules adjacentes de la pluralité de molécules cycliques connectées en série (12) étant capables d'être attirées les unes aux autres par une force de liaison hydrogène et étant agencées d'une manière empilée.

**33.** Composition d'hydrogel (100) selon la revendication 31, sur la molécule linéaire (11), la pluralité de molécules cycliques connectées en série (12) étant agencées d'une manière de têtes à têtes et de queues à queues.

**34.** Lentille d'hydrogel, **caractérisée par le fait qu'**elle comprend : un corps de lentille qui est formé par la composition d'hydrogel (100) selon la revendication 1.

**35.** Lentille d'hydrogel selon la revendication 34, le composé de rotaxane (1) étant distribué sur au moins une surface d'arc concave ou une surface d'arc convexe du corps de lentille pour former une couche de rotaxanes, et le composé de rotaxane (1) étant configuré pour transporter un ingrédient actif.

**36.** Lentille d'hydrogel selon la revendication 35, l'ingrédient actif étant au moins une matière choisie dans un groupe constitué par le menthol, le d-camphre, le d-bornéol, le chlorure de potassium, le chlorhydrate de lysozyme, le glycyrrhizinate dipotassique, l'acide aminocaproïque, l'acide ε-aminocaproïque, l'acide 6-aminohexanoïque, le sulfate de zinc, la vitamine B2, l'acide azulène sulfonique de sodium, le sulfaméthoxazole, le pranoprofène, la naphazoline, la tétrahydrozoline, le chlorobutanol, le chlorhydrate de phényléphrine, la phényléphrine, la vitamine E, le L-aspartate de potassium, le L-aspartate de magnésium, le sulfate de chondroïtine, le méthylsulfate de néostigmine, la vitamine B12, la vitamine B5, la vitamine B6, la vitamine A, la taurine, la diphénhydramine, le chlorphéniraminem, le tranilast, l'acide borique, la glycérine, l'acide hyaluronique, un polyol et l'allantoïne.

**37.** Lentille d'hydrogel selon la revendication 34, un indice de réfraction du corps de lentille étant compris entre 1,315 et 1,598, une transmittance de lumière du corps de lentille n'étant pas inférieure à 90 %, une teneur en eau du corps de lentille étant comprise entre 10 % p/p et 75 % p/p, un angle de contact dynamique du corps de lentille n'étant pas supérieur à 80 degrés, et une élongation du corps de lentille étant comprise entre 250 % et 380 %.

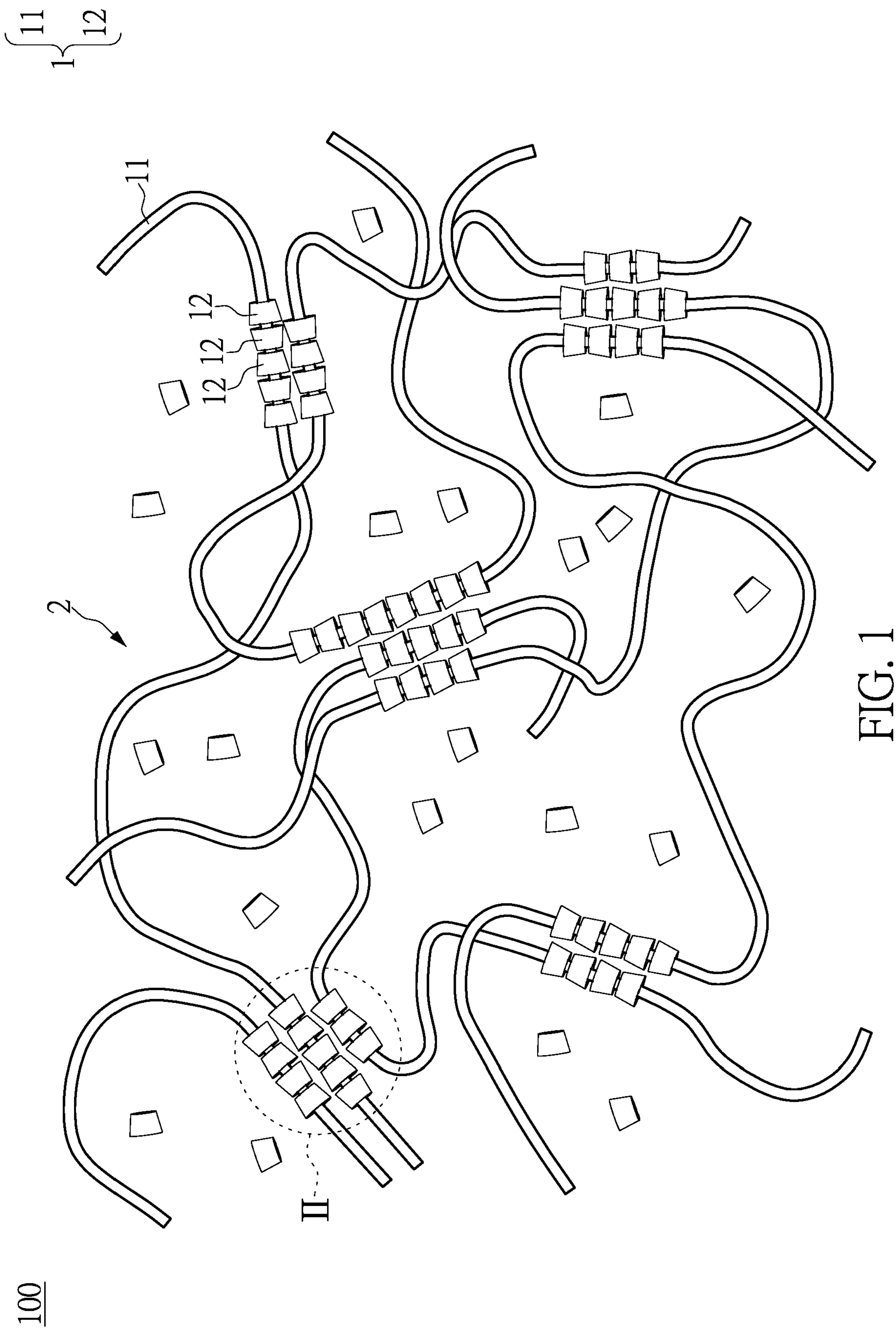


FIG. 1

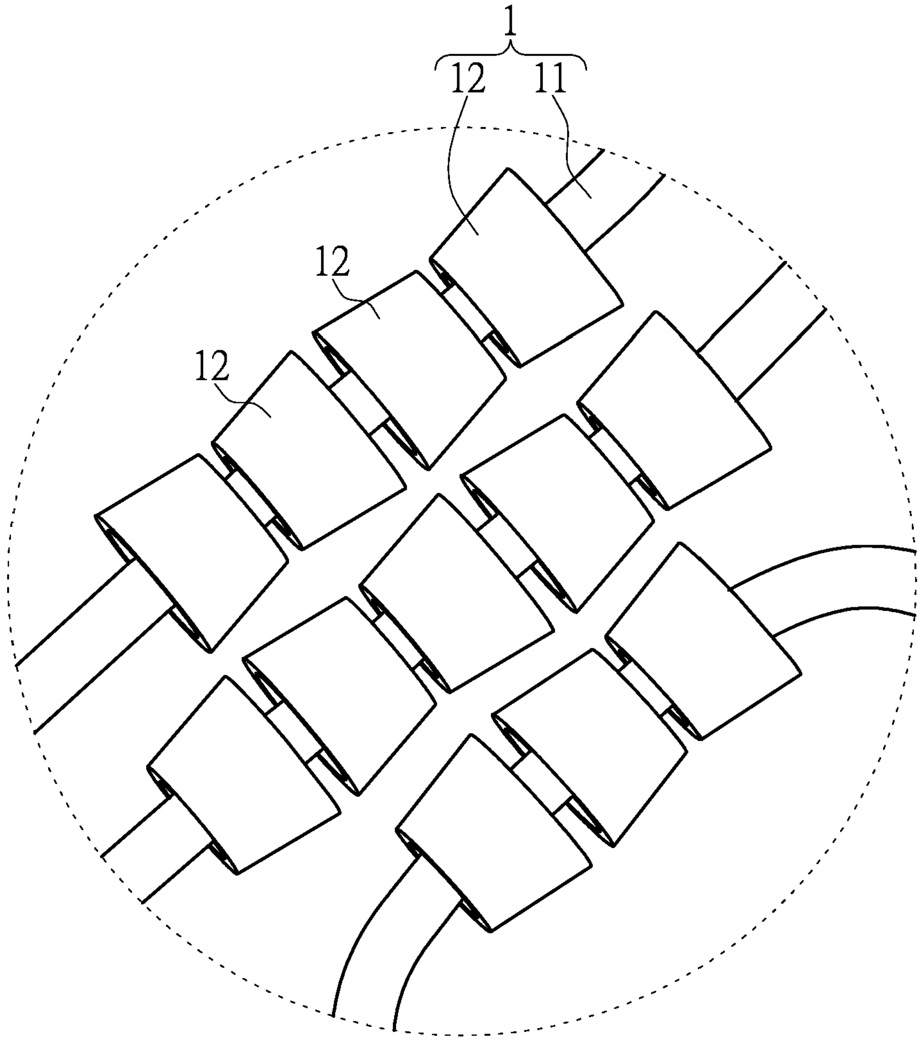


FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- TW 109112349
- TW 109141266
- EP 2947104 A1
- CN 101397347 B

### Non-patent literature cited in the description

- Novel Hydrogels with Excellent Mechanical Performance. **TANAKA Y et al.** Progress In Polymer Science. Pergamon Press, 01 January 2005, vol. 30, 1-9
- *Pol. J. Chem. Tech.,* 2016, vol. 18 (3), 110-116
- *J fluoresc.,* 2007, vol. 17, 265-270
- *J Agric Food Chem.,* 2017, vol. 65 (26), 5404-5412